# EUROPÄISCHE PATENTSCHRIFT

(11) **EP 1 634 875 B1**
(45) Veröffentlichungstag und Bekanntmachung des Hinweises auf die Patenterteilung: **03.01.2007**
(21) Anmeldenummer: 05017346.7
(22) Anmeldetag: 10.08.2005
(51) Int. Cl.: C07D 251/34, C08K 5/3492

(54) **Verfahren zur Herstellung von festem Tris-(2,3-dibrompropyl)-isocyanurat**
Process for the preparation of solid tris-(2,3-dibrompropyl)-isocyanurate
Procedé pour la préparation de tris-(2,3-dibrompropyl)-isocyanurate solide

(30) Priorität: 14.08.2004 DE 102004039490
(43) Veröffentlichungstag der Anmeldung: 15.03.2006
(73) Patentinhaber: DEGUSSA AG, 40474 Düsseldorf (DE)
(72) Erfinder: Werle, Peter, Dr., 63571 Gelnhausen (DE); Schmidt, Manfred, Dr., 63571 Gelnhausen (DE)

(56) Entgegenhaltungen:
- EP-A- 0 443 167
- DE-A1- 2 244 543
- JP-A- 11 228 549
- JP-A- 2000 053 658

## Beschreibung

Die vorliegende Erfindung ist auf ein Verfahren zur Isolierung von Tris-(2,3-dibrompropyl)-isocyanurat gerichtet. Insbesondere wird die eben genannte Verbindung in der Art und Weise isoliert, dass diese aus einer organischen Produktlösung unter Zugabe eines porösen, saugfähigen Materials in eine feste gut filtrierbare Form überführt wird.

Tris(2,3-dibrompropyl)-isocyanurat [im folgenden abgekürzt mit TDPI] ist ein sehr effektives Flammschutzmittel mit hoher thermischer Stabilität, das überwiegend zur Flammfestausrüstung von Polypropylen - daneben auch von Polyethylen und Polystyrol - eingesetzt wird. Vorteile des Produktes sind seine niedrige Viskosität, geringe Verfärbungsneigung und Korrosivität gegenüber den Prozesswerkzeugen während der Verarbeitung.

Das erste Verfahren zur Herstellung des TDPI wurde in der DE2244543 im Jahre 1972 beschrieben. Die Synthese erfolgte durch Bromierung von Triallylisocyanurat [CAS Nr. 1025-15-6] unter Verwendung von Dichlormethan als Lösungsmittel. Nach erfolgter Bromzugabe wurde das Reaktionsprodukt durch Zusatz von Petrolether ausgefällt, filtriert, der Filterkuchen mit MeOH verrieben und mit Wasser gewaschen. Diese Verfahrensweise - Reaktion in einem vorzugsweise chlorierten Lösungsmittel und Ausfällung des TDPI durch Zusatz eines Nichtlösers - wird auch in vielen neueren Patentanmeldungen beschrieben.

In der JP 2000053658 wird Triallylisocyanurat in Dichlormethan bromiert und Methanol als Fällmittel eingesetzt. Die JP 1999228549 beansprucht eine Mischung aus Heptan und Dichlormethan als Reaktionsmedium und weiteres Heptan zur Fällung.

In der JP 2000053658 werden neben Dichlormethan Reaktionslösungsmittel wie 1,1,1-Trichlorethan, Chloroform, Kohlenstofftetrachlorid, Ethylendichlorid, Dibrommethan und andere genannt. Als Fällungsmittel werden u.a. aufgeführt: Methanol, Ethanol, i-Propanol, Pentan, Hexan, Cyclohexan. Um überschüssiges Brom zu entfernen (Farbreduzierung) wird noch eine Behandlung der Reaktionslösung mit einer wässrigen Hydrazinlösung durchgeführt.

Bei all diesen Verfahrensvarianten treten grundlegende Probleme auf, die einmal mit den physikalischen Eigenschaften des TDPI zusammenhängen und weiterhin mit den chem./phys. Eigenschaften der verwendeten Fällungsmittel. Obwohl das TDPI einen Schmelzpunkt > 100°C aufweist, neigt es dazu, bei der Fällung aus Lösungen durch Zusatz eines Nichtlösungsmittels in flüssiger Form auszufallen. Diese flüssige Phase erstarrt nach einiger Zeit und geht in festes Produkt in kompakter Form über. Dieses Verhalten ist verfahrenstechnisch nur durch den Einsatz schwerster Rührorgane wie z.B. durch Schneckenkneter zu bewältigen. Normale Rührwerke wie sie in Rührwerkskesseln eingesetzt werden, sind überfordert und schalten ab. Die Ausfällung des TDPI in einer rührfähigen kristallinen und filtrierbaren Form unter Umgehung der flüssigen Phase erfordert ein sehr differenziertes Verfahren der Fällmittelzugabe.

Eine zweite Schwierigkeit liegt darin, dass nur noch sehr wenige chlorierte Kohlenwasserstoffe technisch eingesetzt werden dürfen. Dazu gehört Dichlormethan. Bei Verwendung des Systems Dichlormethan als Reaktionslösemittel und Methanol als Fällmittel ist eine destillative Trennung nicht mehr möglich, da sich ein azeotropes System der Zusammensetzung 93 % CH2Cl2; 7 % CH3OH und einem Siedepunkt von 38 °C ausbildet. Eine solche Mischung kann aber bei Folgeansätzen nicht als Lösungsmittel verwendet werden, da das Methanol mit Brom reagiert und hochkorrosiven Bromwasserstoff freisetzt.
Auch Benzine oder Heptan reagieren mit Brom, so dass eine unverzichtbare Forderung an den Verfahrensprozess die saubere destillative Abtrennung des Fällmittels vom Dichlormethan sein muss. Erfahrungsgemäß sollte der Gehalt an Fällmittel im Dichlormethan 0,1 % nicht überschreiten.

Weiterhin zeigt sich, dass sich im Fällungsmittel bei mehrfacher Verwendung Nebenprodukte anreichern, die beim Stehen lassen zu klebrigen Abscheidungen führen und eine mehrmalige Reinigung der Behältnisse erfordern, sowie eine Destillation auch des Fällungsmittels.

Ein Verfahrensprozess, der die oben geschilderten Problematiken berücksichtigt, sollte also wie folgt aussehen:
- Umsetzung von Triallylisocyanurat mit Brom in Dichlormethan
- Zusatz eines Fällmittels, dass mit Dichlormethan kein Azeotrop bildet
- Kristallisation des TDPI
- Filtration und Waschen (mit Fällungsmittel)
- Trocknung
- Trennung des Dichlormethans vom Fällmittel mittels mehrstufiger Destillation
- Je nach Verunreinigungsgrad Destillation des Fällmittels
- Entsorgung von Nebenprodukten

Es zeigt sich somit, dass ein verhältnismäßig hoher Aufwand für die Herstellung von TDPI im großen Maßstab erforderlich ist.

Aufgabe der vorliegende Erfindung war es deshalb, ein weiteres Verfahren zur Herstellung von festem TDPI anzugeben, was die Nachteile der Verfahren des Standes der Technik in möglichst effizienter und bei der Produktion im großen Maßstab kostengünstiger Art und Weise zu überwinden hilft. Insbesondere sollte das Verfahren in der technischen Synthese vom ökonomischen und ökologischen Standpunkt aus gesehen Vorteile bieten. Dies sollte auch gerade für die Tatsache einer überlegenen Prozessführung gelten.

Diese Aufgabe wird anspruchsgemäß gelöst. Anspruch 1 ist auf ein Verfahren zur Generierung von festem Tris(2,3-dibrompropyl)-isocyanurat gerichtet. Abhängige Unteransprüchen 2 bis 6 betreffen bevorzugte Ausführungsformen. Ansprüche 7 und 8 betreffen das so hergestellten Produkt und Anspruch 9 dessen vorteilhafte Verwendung.

Dadurch, dass man in einem Verfahren zur Herstellung von festen Präzipitaten von Tris-(2,3-dibrompropyl)-isocyanurat, die besagte Verbindung in einem organischen Lösungsmittel mit einem unter den gegebenen Umständen festen porösen Adsorptionsmittel mit einer BET-Oberfläche von 50 - 700 m²/g kontaktiert und isoliert, gelangt man in völlig überraschender dafür aber nicht minder vorteilhafter Art und Weise zur Lösung der genannten Aufgaben. Vorteilhaft ist vor allem, dass sich das schwer kristallisierbare TPDI in quantitativer Ausbeute in festes Material überführen lässt. Darüber hinaus weisen die so hergestellten Präzipitate keine Klebrigkeit auf und lassen sich sehr gut vom Lösungsmittel befreien, was an der überraschend einsetzenden Verfestigung des TDPIs auf dem Adsorptionsmittel liegt. Das erfindungsgemäße Verfahren vermeidet also die Handhabung von flüssigen Fällungsmitteln und die sich daraus ergebenden eingangs geschilderten Folgeprobleme. Gleichfalls lassen sich durch Einsatz des porösen Adsorptiionsmittels Konzentrationen von 80% und mehr an TPDI im Adsorbens erreichen. Obgleich das TPDI sich auf dem Adsorbens verfestigt, und somit nach kurzer Zeit zwei feste Phasen vorliegen, ist wiederum überraschenderweise keinerlei Migration oder Separation festzustellen.

Als poröse Adsorptionsmittel zur Initiierung der Adsorption und Bildung der festen Präzipitate von TPDI können im Prinzip dem Fachmann zur Verfügung stehende Materialien herangezogen werden, sofern sie unter den gegebenen Umständen eine feste Konsistenz aufweisen. Vorteilhaft sind solche, die hinsichtlich der eingesetzten Reagenzien und Lösungsmittel inert reagieren. Im Einzelnen sind Materialien vorteilhaft, welche ausgewählt sind aus der Gruppe bestehend aus: Polymergranulate, Kieselsäure, Silikate.

Unter Polymergranulaten sind mikroporöse polymere Produkte zu verstehen, die aus handelsüblichen Polymeren wie PP, PE, PA hergestellt werden. Die mikroporösen Strukturen wirken wie kleine Schwämme und sind in der Lage, ein Mehrfaches ihres Gewichtes an Flüssigkeiten durch kapillare Adsorption aufzunehmen. Auch in beladenem Zustand bleibt das System überraschend trocken und fließfähig. Hergestellt werden derartige Granulate z.B. von der Membrana GmbH/Obernburg unter dem Handelsnamen Accurel^{®}. Besonders vorteilhaft ist die Verwendung von granuliertem, mikroporösen Polypropylen in diesem Zusammenhang, da das TPDI überwiegend als flammhemmendes Mittel in aus Polypropylen gefertigten Artikeln eingesetzt wird und damit erfindungsgemäß kein weiterer Fremdstoff zugesetzt werden muss.

Unter Kieselsäuren sind Auto-Kondensationsprodukte der ortho-Kieselsäuren (H₄SiO₄) mit der allg. Formel (SiO₂)ₘ x H₂O zu verstehen. Sie werden großtechnisch durch Fällen von Alkalisilicatlösungen mit Säuren hergestellt und daher als Fällungskieselsäuren bezeichnet. Daneben können hochdisperse Produkte auch durch Verbrennung von SiCl₄ im Knallgasstrom gewonnen werden. Für die Absorption des TPDI werden vorzugsweise Fällungskieselsäuren eingesetzt. Diese Produkte weisen BET-Oberflächen von 50 - 700 m²/g auf. Die vorteilhaft einsetzbaren Kieselsäuren können den Produktkatalogen z.B. der Firma Degussa entnommen werden.

Silikate sind Metallsalze der ortho-Kieselsäure und deren Kondensationsprodukte. Im vorliegenden Fall kann bevorzugt pulverförmiges Calciumsilikat eingesetzt werden.

Die eingesetzten Adsorptionsmittel sollen unter den gegebenen Umständen eine feste Konsistenz aufweisen, d.h. dass die strukturelle Integrität der Adsorptionsmittel zumindest über die Zeitdauer der Adsorption und Isolierung gewährleistet sein muss, damit wie erfindungsgemäß gefordert feste Präzipitate erhalten werden können. Da die Adsorption vorteilhafterweise im Bereich von 0 - 100°C, vorzugsweise 15 - 80°C und ganz besonders bevorzugt 20 - 50°C durchgeführt wird, sollte die strukturelle Integrität der Adsorptionsmittel über den oben genannten Zeitraum bei mindestens 100°C gewährleistet sein.

Wie angedeutet sollte die BET-Oberfläche der Adsorptionsmittel im Bereich von 50 - 700 m²/g liegen, um einen wirtschaftlich sinnvollen Einsatz der Adsorptionsmittel zu gewährleisten. Bevorzugt kann die BET-Oberfläche zwischen 60 - 500 m²/g und besonders bevorzugt zwischen 80 - 200 m²/g betragen.

Für das Verfahren essenziell ist das Vorhandensein von Poren in dem zugefügten Adsorptionsmittel. Die Poren sollten dabei einen solchen mittleren Porendurchmesser aufweisen, der das möglichst effiziente Eindringen von TPDI in das Adsorbens gestattet. Insbesondere vorteilhaft sind Materialien, die einen mittleren Porendurchmesser von 1 - 100 µm, vorzugsweise 3 - 50 µm und ganz besonders bevorzugt 5 - 20 µm besitzen.Der Porendurchmesser wird nach DIN 66133 bzw. DIN 66134 bestimmt.

Als organisches Lösungsmittel können bei der Adsorption solche eingesetzt werden, die gegen die vorhandenen Reagenzien inert sind. Da das Herstellungsverfahren vorteilhafterweise über eine radikalische Bromierung von Triallylisocyanurat vonstatten geht, sind solche organischen Lösungsmittel zu bevorzugen, die auch für radikalische Bromierungen eingesetzt werden können. Insbesondere sind dies Lösungsmittel ausgewählt aus der Gruppe bestehend aus: Dichlormethan, Tetrachlorethan, Essigsäure, Tetrachlorkohlenstoff, Chloroform oder Mischungen derselben.

Im Einzelnen geht man beim erfindungsgemäßen Verfahren vorteilhafterweise so vor, dass zu der aus einem Herstellungsprozess stammenden fertigen Redaktionslösung, welche das TPDI enthält, das poröse Adsorptionsmittel zur Fällung hinzugegeben wird. Prinzipiell ist auch die umgekehrte Vorgehensweise denkbar. Dies geschieht im wesentlichen bei Temperaturen, die die effiziente Isolation des TPDIs gestatten, insbesondere im oben angegebenen Temperaturrahmen. Nach Zugabe des Adsorptionsmittels wird in der Regel noch etwas nachgerührt, um die Adsorption des TPDI auf dem Adsorptionsmittel zu vervollständigen. Anschließend kann die Isolierung und Trocknung erfolgen. Zur Isolierung kann das Reaktionslösungsmittel abdestilliert und das zurückbleibende feste Präzipitat erfindungsgemäß weiterverarbeitet werden. Gegenstand der vorliegende Erfindung ist ebenfalls ein Feststoff, bei dem dieser im wesentlichen aus einem festen Präzipitat aus Tris-(2,3-dibrompropyl)-isocyanurat, welches adsorptiv gebunden auf dem festen porösen Adsorptionsmittel vorliegt, besteht.

Einen abschließenden Aspekt der vorliegende Erfindung bildet die Tatsache der Verwendung des erfindungsgemäßen Feststoffes zur Flammschutzausrüstung von Kunstoffprodukten. Der Feststoff kann in einer bevorzugten Ausführungsform so, wie er nach Isolation und Trocknung erhältlich ist, in den zu schützenden Artikel eingearbeitet werden. Als solche Artikel kommen im wesentlichen Kunststoffe und die aus ihnen herstellbaren Gegenstände in Frage. Vorteilhaft werden der erfindungsgemäße Feststoff zur Flammeschutzausrüstung von Polypropylen, Polyethylen und Polystyrol und den daraus hergestellten Produkten eingesetzt.

Wie angegeben kann durch Anwendung des erfindungsgemäßen Verfahrens das ansonsten schwer in fester Form isolierbare TPDI in praktisch quantitativer Ausbeute und Reinheit durch Adsorption auf porösen Adsorptionsmitteln in einer solchen Form gewonnen werden, die einerseits eine vorzügliche Isolierung und andererseits deren einfache Weiterverarbeitung in die auszurüstenden Gegenstände gestatten. Die Tatsache der guten Handhabbarkeit des Produkts und der schnellen und einfachen Prozessführung gerade auch im großen Maßstab bilden essentielle Vorteile, welche das erfindungsgemäße Verfahren, den hergestellten Feststoff und dessen Verwendung vor dem Hintergrund des Standes der Technik als nicht naheliegend erscheinen lassen.

### Beispiel 1

In einem Kolben werden 60 g Triallylisocyanurat in 150 ml Dichlormethan vorgelegt und 114 g Brom so zugegeben, daß gelinder Rückfluß eintritt. Man rührt 1 h nach bis die Bromfarbe verschwunden ist und trägt 170 g poröses Polypropylen (z.B. Accurel^{®} MP 100 oder MP 1000) ein. Durch Anlegen eines schwachen Vakuums wird bei ca. 40 °C das Dichlormethan ausgetrieben und die Präparation mit 50 Gew.% TDPI hinterbleibt als praktisch farbloses, freifließendes Granulat ohne Ausbildung klebriger Beläge.

Alternativ kann auch die Reaktionslösung zu vorgelegtem Polymer gegeben werden auch unter gleichzeitigem Abdampfen des Lösungsmittels.

### Beispiel 2

Eine bromierte Triallylisocyanuratlösung, hergestellt gemäß Beispiel 1, wird auf eine vorgelegte Fällungskieselsäure (z.B. Sipernat 50)* gegeben und das Lösungsmittel destillativ entfernt.

Es hinterbleibt eine 70 Gew.% enthaltenden TDPI-Präparation, die nicht klebt und freifliessend ist.
* Herstelller Degussa AG

## Patentansprüche

1. Verfahren zur Herstellung von festen Präzipitaten von Tris-(2,3-dibrompropyl)-isocyanurat **dadurch gekennzeichnet, dass**
man besagte Verbindung in einem organischen Lösungsmittel mit einem unter den gegebenen Umständen festen porösen Adsorptionsmittel mit einer BET-Oberfläche von 50 - 700 m²/g kontaktiert und isoliert.

2. Verfahren nach Anspruch 1,
**dadurch gekennzeichnet, dass**
das poröse Adsorptionsmittel ausgewählt ist aus der Gruppe bestehend aus: Kieselsäure, Polymergranulate, Silikate.

3. Verfahren nach Anspruch 1 und/oder 2,
**dadurch gekennzeichnet, dass**
das Material im Temperaturbereich von ≥100 °C eine feste Konsistenz aufweist.

4. Verfahren nach einem oder mehreren der vorhergehenden Ansprüchen,
**dadurch gekennzeichnet, dass**
das Material einen mittleren Porendurchmesser von 1 - 100 µm aufweist.

5. Verfahren nach einem oder mehreren der vorhergehenden Ansprüche,
**dadurch gekennzeichnet, dass**
als organisches Lösungsmittel eines aus der Gruppe bestehend aus: Dichlormethan, Tetrachlorethan, Essigsäure, Tetrachlorkohlenstoff, Chloroform oder Mischungen derselben eingesetzt wird.

6. Feststoff erhältlich nach einem oder mehreren der vorhergehenden Ansprüche, wobei das Tris-(2,3-dibrompropyl)-isocyanurat adsorptiv gebunden auf dem festen porösen Adsorptionsmittel vorliegt.

7. Verwendung des Feststoffes nach Anspruch 6 zur Flammschutzausrüstung von Kunstoffprodukten.

## Claims

1. Process for preparation of solid precipitates of tris(2,3-dibromopropyl) isocyanurate,
**characterized in that**
the said compound is brought into contact, in an organic solvent, with an adsorbent which is porous and solid under the present circumstances and has a BET surface area of from 50 to 700 m²/g, and is isolated.

2. Process according to Claim 1,
**characterized in that**
the porous adsorbent has been selected from the group consisting of: silica, polymer granules, silicates.

3. Process according to Claim 1 and/or 2,
**characterized in that**
the material has solid consistency in the temperature range ≥ 100°C.

4. Process according to one or more of the preceding claims,
**characterized in that**
the material has an average pore diameter of from 1 to 100 µm.

5. Process according to one or more of the preceding claims,
**characterized in that**
the organic solvent used comprises a solvent from the group consisting of: dichloromethane, tetrachloromethane, acetic acid, carbon tetrachloride, chloroform or a mixture of the same.

6. Solid obtainable according to one or more of the preceding claims, wherein the tris(2,3-dibromopropyl) isocyanurate is bound by adsorption on the solid porous adsorbent.

7. Use of the solid according to Claim 6 for providing flame retardancy to plastics products.

## Revendications

1. Procédé pour la préparation de précipités solides de tris-(2,3-dibromopropyl)-isocyanurate, **caractérisé en ce qu'**on met en contact ledit composé dans un solvant organique avec un agent d'adsorption qui est poreux et solide dans les conditions données, présentant une surface BET de 50 - 700 m²/g et on l'isole.

2. Procédé selon la revendication 1, **caractérisé en ce que** l'agent d'adsorption poreux est choisi dans le groupe constitué par : la silice, les granulats de polymères, les silicates.

3. Procédé selon la revendication 1 et/ou 2, **caractérisé en ce que** le matériau présente une consistance solide dans la plage de température ≥100°C.

4. Procédé selon l'une ou plusieurs des revendications précédentes, **caractérisé en ce que** le matériau présente un diamètre moyen des pores de 1-100 µm.

5. Procédé selon l'une ou plusieurs des revendications précédentes, **caractérisé en ce qu'**on utilise comme solvant organique un solvant du groupe formé par : le dichlorométhane, le tétrachloroéthane, l'acide acétique, le tétrachlorure de carbone, le chloroforme ou des mélanges de ceux-ci.

6. Solide pouvant être obtenu selon l'une ou plusieurs des revendications précédentes, le tris-(2,3-dibromopropyl)-isocyanurate se trouvant sous une forme liée par adsorption sur l'adsorbant poreux solide.

7. Utilisation du solide selon la revendication 6 pour l'apprêt de protection contre les flammes de produits en matériau synthétique.
